# EUROPEAN PATENT APPLICATION

(11) **EP 3 907 274 A1**
(43) Date of publication of application: **10.11.2021**
(21) Application number: 19718771.9
(22) Date of filing: 04.01.2019
(51) Int. Cl.: C12M 3/00, C12M 1/12, C12M 1/00, C12M 1/42

(54) **BIOREACTOR AND METHOD FOR THE PRODUCTION OF ADHERENT CELL CULTURES EMPLOYING SAID BIOREACTOR**

(71) Applicant: Biotech Foods S.L., 20018 San Sebastián (ES)
(72) Inventor: VILA JUÁREZ, Mercedes, 20018 San Sebastián (ES)
(74) Representative: Balder IP Law, S.L.
(86) International application number: PCT/ES2019/070006
(87) International publication number: WO 2020/141236

(57) **Abstract**

Bioreactor and production process of adherent cell cultures that uses the bioreactor. The bioreactor (1) comprises a recipient (2) with a cover (3), and further comprises internally a bracket (4) from which protrudes at least one tray (5) that supports a three-dimensional polymer-based element (6) mounted on it, such that when the bioreactor (1) is filled with a culture medium the at least one tray (5) and the three-dimensional polymer-based element (6) are surrounded by the culture medium.

The process comprises the following steps:
- the bracket (4), with the at least one tray (5) of the bioreactor (1), with a three-dimensional polymer-based element (6) is immersed in the culture medium,
- cells that are desired to be planted on the three-dimensional polymer-based element (6) are inoculated from outside the bioreactor (1), and
- the culture is left in the bioreactor (1) so that the cellular proliferation is produced.

## Description

### Field of the invention

This invention relates to a bioreactor (also called a fermenter), of special application in biochemical processes wherein microbial or cellular systems are used for the production of biological products. It also refers to a production process of adherent cell cultures, especially for the formation of tissues and cultivated flesh that uses said bioreactor.

### Background of the invention

Bioreactors have multiple applications, such as the food and fermentation industries, waste treatment, and many biomedical facilities.

A fermenter is a tank inside of which organic reactions take place, providing an environment suitable for various micro-organisms under the required conditions. It must permit the control of the temperature by heat or cold, to control the pH, and the ranges of aeration, nutrient content, etc.

The common bioreactors are generally containers or systems that maintain a biologically active atmosphere. In some cases, a bioreactor is a container wherein chemical process takes place that involves organisms or biochemically active substances derived from such organisms. This process can be aerobic or anaerobic. These bioreactors are commonly cylindrical, ranging in size from a few millilitres to cubic meters, and are usually made of stainless steel.

A bioreactor can also be a device or system used to grow cells or tissues in cell culture operations. These devices are under development for use in tissue engineering.

In general terms, a bioreactor seeks to maintain certain favourable environmental conditions (pH, temperature, oxygen concentration, etc.) for the organism or chemical substance that is being cultivated.

The large-scale industrial fermenters are made of stainless steel. A fermenter of this type is a cylinder closed top and bottom, wherein a number of tubes and valves have been adapted. Other materials are also used, and there is a tendency towards single-use plastics; however, the high costs of consumables are only justified if the resulting product is of high market value (for example: antibodies, vaccines, therapeutic proteins, etc. obtained through the culture of mammalian and insect cells).

A good fermenter design must meet two fundamental requirements: maintaining a homogeneous medium without dead zones and, at the same time, transferring oxygen to the environment using the minimum energy possible.

The design of a fermenter should consider the following aspects:
- mixture and flow patterns
- geometric configuration and type of reactor
- transfer of oxygen throughout the tank
- minimal energy consumption.

The types of fermenters available on the market present a series of components and common characteristics:
- the tank must be designed to operate aseptically for many days
- minimal energy consumption
- include a pH control system
- sampling system
- an adequate system of aeration and agitation to cover the metabolic needs of micro-organisms
- temperature control
- minimal evaporation
- it must be versatile (various applications or processes)
- internal tank surfaces must be smooth
- inexpensive materials
- adequate service of spare parts for the fermenter
- type of antifoam.

The use of bioreactors is widespread for the cultivation of micro-organisms (bacteria, fungi, cells, etc.), both at a laboratory scale as well as at an industrial scale. Its design is usually for a tank agitated by means of mechanical devices, either by rotating blades or by a bubbling column. And the micro-organism cultures are made in suspension. The cell suspensions consist of free cells and cell aggregates that are distributed in an environment in movement. This becomes a problem when the cells that are to be grown are cells referred to as "adherents". These cells, since they are the muscle cells or epithelial cells, need a surface to be able to grow and proliferate, such that the solutions provided by the bioreactors that maintain the cells in suspension are not appropriate for them. It is only possible to grow micro-organisms in the bioreactors when they are able to proliferate in suspension

To be able to grow these adherent cells, various proposals have been proposed and marketed:
- Fixed-bed bioreactors, wherein the surface available for these cells to proliferate colonising the area available at the bottom of the vessel, is very limited.
- Static culture flasks within a CO2 oven; in the same way they remain only slightly productive and limited by the available two-dimensional surface.

The most significant advance to growing adherent cells has been the proposal to use microspheres suspended in the solution of the traditional fermenters/bioreactors, in such a way as to achieve increased specific surface area that these cells can find within the suspension and where they can anchor themselves to grow and proliferate.

Another example is the iCELLis® bioreactor, a fixed bed system for the growth of adherent cells.

### Summary of the invention

The object of this invention is, therefore, to provide a bioreactor that permits growing adherent cells in a simple and effective way, so that it is productive on a large scale, in particular for the production of tissues and cultivated flesh.

The invention provides a bioreactor, which comprises a container with a cover, and that further comprises an internal bracket from which protrudes at least one tray that supports a three-dimensional polymer-based element mounted on it, so that when the bioreactor is filled with a culture medium the at least one tray and the three-dimensional polymer-based element are surrounded by the culture medium.

The invention also provides a production process of adherent cell cultures that uses said bioreactor, and that comprises the following steps:
- the bracket, with the at least one tray of the bioreactor, with a three-dimensional polymer-based element,is immersed in the culture medium,
- cells that are desired to be planted on the three-dimensional polymer-based element are inoculated from outside the bioreactor, and
- the culture is left in the bioreactor so that it produces the cellular proliferation.

The configuration of the bioreactor permits that the proliferated adherent cells achieve their growth functions of extracellular matrix and tissue formation in a manner that is more similar to the in vivo processes and that the corresponding process is more productive. In this way, the formed tissues have a high protein value.

Other advantageous embodiments of the invention are outlined in the dependant claims.

### Brief description of the figures

An illustrative embodiment is presented below, which is not limitative in any sense, of the purpose of this invention, with reference to the accompanying drawings wherein:
Figure 1 shows a view of the invention bioreactor that shows the bracket and the trays.
Figure 2 shows the assembly formed by the bracket, trays, rings, and the three-dimensional polymer-based elements of the bioreactor of the invention.
Figure 3 shows the assembly of Figure 2, to which air bubble generators have been added underneath each tray.

### Detailed description of the invention

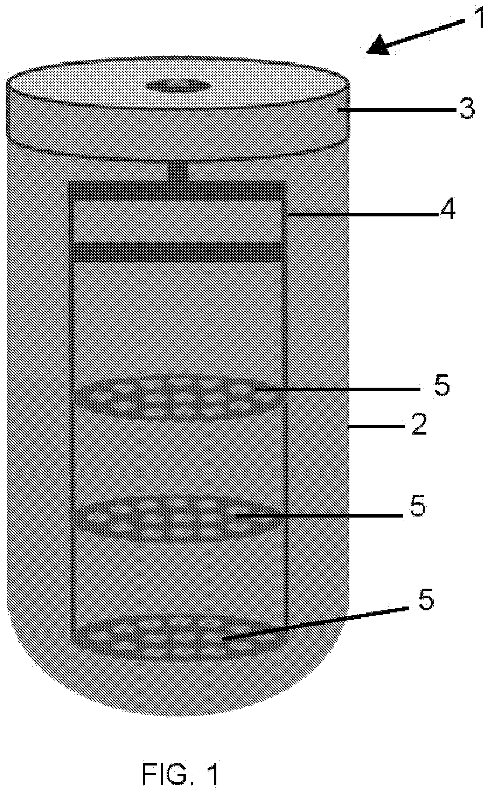

Figure 1 shows a view of a bioreactor 1 of the invention obtained from a commercial or traditional bioreactor. The conventional bioreactor comprises a container 2 with a cover 3. A bracket 4 has also been included inside the bioreactor 1 of the invention on which the trays 5 can be installed. In the embodiment of Figures 1, 2 and 3, the bioreactor 1 has three trays 5 that can be made of stainless steel. The number of trays 5 may increase or decrease depending on the needs and the size of the bioreactor 1.

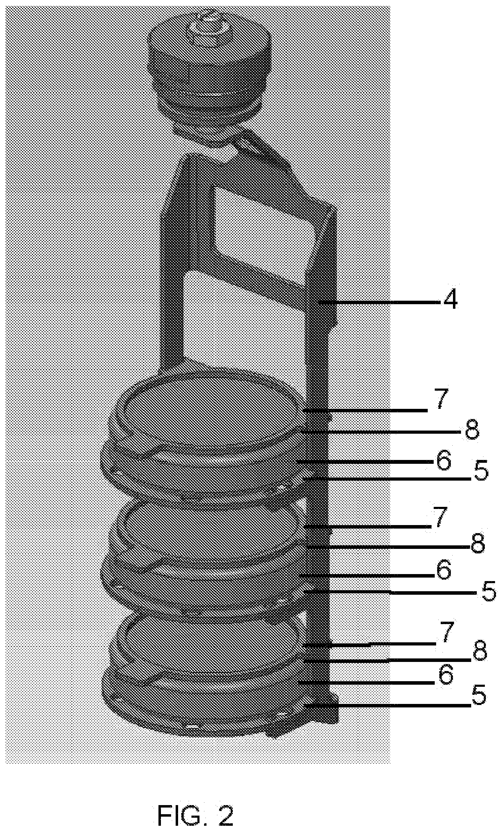

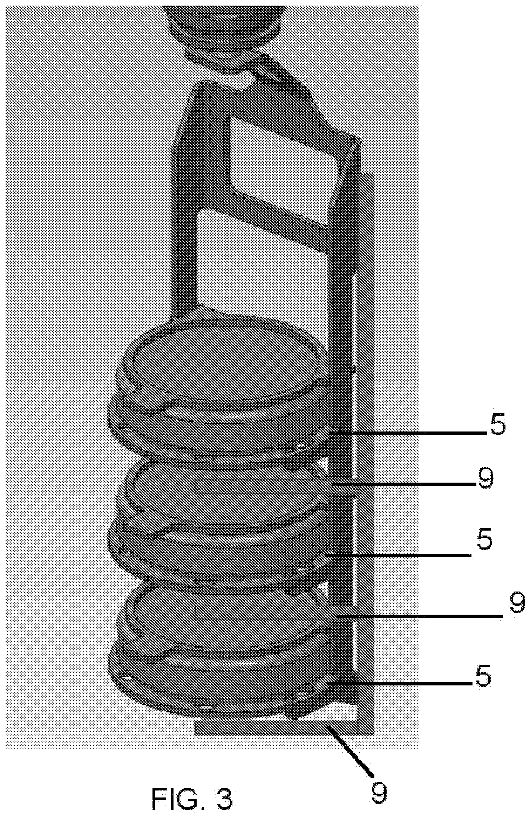

In Figures 2 and 3, it is noted that in the bioreactor 1 of the invention, each tray 5 supports a three-dimensional polymer-based element 6 mounted on it, that is to say, these trays 5 represent places of support for three-dimensional elements 6 (three-dimensional arrays) that act as growth "scaffolds" for the adherent cells.

These "scaffolds" may be hydrogel type three-dimensional polymer-based structures 6, on which the cells can be sown by inoculation with a needle from outside the bioreactor 1 and left in cultivation until the desired proliferation is achieved.

When the bioreactor 1 is filled with a culture medium, the trays 5 and the three-dimensional polymer-based elements 6 are surrounded by the culture medium.

The bracket 4 can be found suspended from the cover 3 of the bioreactor 1, such that when the bioreactor 1 is closed, the trays 5 and the three-dimensional polymer-based elements 6 are submerged in the culture medium. In one embodiment, the bracket 4 can protrude perpendicularly from the cover 3.

The bracket 4 may also be supported from the base of the bioreactor 1, independent of the cover 3.

The trays 5 may be drilled through with a series of holes, to permit a better flow of fluid throughout the whole bioreactor 1.

In Figures 2 and 3 it can be seen that the bioreactor 1 may include retaining rings 7 of the three-dimensional polymer-based elements 6 ("scaffolds") that also protrude from the bracket 4 in a position parallel with respect to a corresponding tray 5, so that the three-dimensional polymer-based element 6 is located between the tray 5 and the corresponding ring 7.

The bracket 4 may include various profiles or support rods on which the trays 5 and rings 7 are supported. It is possible to include some electrical contacts 8 on these profiles that can be fed with alternating or direct current. This alternating or direct current, which may be in the order of micro-amperes, facilitates and increases cell proliferation.

This increased cellular proliferation is due to the fact that, on polarised surfaces and environments, the proteins are more concentrated by electrostatic attraction and the cells have more nutrients available for their metabolic activities. The tests carried out have permitted increases in such proliferation of 7 times with respect to the system without the stimulus of direct current.

The bioreactor 1 can also include an air bubble generator 9 underneath each tray 5. The capacity to modify the size and frequency of the bubbling air by the introduction of bubble generators 9 ("spargers") below each tray 5, exerts a mechanical stimulus on the "scaffolds" with the pressure of these tiny bubbles. This modification is oriented to simulate the contraction/extension movements of the muscular cells to promote the similarities with in vivo proliferation.

The production process for the cell cultures that uses a bioreactor 1, comprises the following steps:
- the bracket 4, with the at least one tray 5 of the bioreactor 1. with a three-dimensional polymer-based element 6 is immersed in the culture medium,
- cells that are desired to be planted on the three-dimensional polymer-based element 6 are inoculated from outside the bioreactor 1, and
- the culture is left in the bioreactor 1 so that it produces the cellular proliferation.

This process can further comprise a stage of cell proliferation stimulation consisting in feeding alternating or direct current to the electrical contacts 8 on the bracket 4 of the bioreactor 1.

Also, this process can further comprise a stage of cell proliferation stimulation consisting in the production of air bubbles underneath each tray 5 by means of the air bubble generators 9.

The invention is of particular application to the cultivation of animal muscle cells for the production of cultivated flesh but is not limited only to this application nor this type of adherent cells.

Although some embodiments of the invention have been described and represented, it is clear that modifications may be introduced to them within the scope of the same, and that this should not be considered limited to these embodiments, but only to the content of the following claims.

## Claims

1. Bioreactor (1) that comprises a recipient (2) with a cover (3), **characterised in that** it also comprises internally a bracket (4) from which protrudes at least one tray (5) that supports a three-dimensional polymer-based element (6) mounted on it, such that when the bioreactor (1) is filled with a culture medium the at least one tray (5) and the three-dimensional polymer-based element (6) are surrounded by the culture medium.

2. Bioreactor (1), according to claim 1, wherein the bracket (4) is suspended from the cover (3).

3. Bioreactor (1) according to claim 2, wherein the bracket (4) protrudes perpendicularly from the cover (3).

4. Bioreactor (1) according to claim 1, wherein the bracket (4) is supported on the base of the bioreactor (1).

5. Bioreactor (1) according to any of the preceding claims, wherein the at least one tray (5) is drilled by a series of through holes.

6. Bioreactor (1) according to any of the preceding claims, that further comprises at least one ring (7) protruding from the bracket (4), so that each ring (7) is arranged in parallel to a corresponding tray (5), being the three-dimensional polymer-based element (6) located between the corresponding tray (5) and the corresponding ring (7).

7. Bioreactor (1) according to any of the preceding claims, where the bracket (4) comprises a series of electrical contacts (8) able to be fed with direct current.

8. Bioreactor (1) according to any of the preceding claims, that further comprises an air bubble generator (9) underneath each tray (5).

9. Production process of adherent cell cultures that uses a bioreactor (1) according to claim 8, that comprises the following steps:
- the bracket (4), with the at least one tray (5) of the bioreactor (1), with a three-dimensional polymer-based element (6) is immersed in the culture medium,
- the cells that are desired to be planted on the three-dimensional polymer-based element (6) are inoculated from outside the bioreactor (1), and
- the culture is left in the bioreactor (1) so that the cellular proliferation is produced.

10. Production process of adherent cell cultures, according to claim 9, that further comprises a stage of stimulation of cell proliferation consisting in feeding direct current to the electrical contacts (8) of the bracket (4) in the bioreactor (1).

11. Production process of adherent cell cultures, according to claim 9 or 10, that further comprises a stage of cell proliferation stimulation consisting in the production of air bubbles underneath each tray (5) by means of the air bubble generators (9).

## Amended claims

### Amended claims under Art. 19.1 PCT

1. Bioreactor (1) that comprises a recipient (2) with a cover (3), also comprising internally a bracket (4) from which protrudes at least one tray (5) that supports a three-dimensional polymer-based element (6) mounted on it, such that when the bioreactor (1) is filled with a culture medium the at least one tray (5) and the three-dimensional polymer-based element (6) are surrounded by the culture medium, **characterized in that** the bracket (4) comprises a series of electrical contacts (8) able to be fed with alternating current or with direct current.

2. Bioreactor (1), according to claim 1, wherein the bracket (4) is suspended from the cover (3).

3. Bioreactor (1) according to claim 2, wherein the bracket (4) protrudes perpendicularly from the cover (3).

4. Bioreactor (1) according to claim 1, wherein the bracket (4) is supported on the base of the bioreactor (1).

5. Bioreactor (1) according to any of the preceding claims, wherein the at least one tray (5) is drilled by a series of through holes.

6. Bioreactor (1) according to any of the preceding claims, that further comprises at least one ring (7) protruding from the bracket (4), so that each ring (7) is arranged in parallel to a corresponding tray (5), being the three-dimensional polymer-based element (6) located between the corresponding tray (5) and the corresponding ring (7).

7. Bioreactor (1) according to any of the preceding claims, that further comprises an air bubble generator (9) underneath each tray (5).

8. Production process of adherent cell cultures that uses a bioreactor (1) according to claim 7, that comprises the following steps:
- the bracket (4), with the at least one tray (5) of the bioreactor (1), with a three-dimensional polymer-based element (6) is immersed in the culture medium,
- the cells that are desired to be planted on the three-dimensional polymer-based element (6) are inoculated from outside the bioreactor (1),
- the culture is left in the bioreactor (1) so that the cellular proliferation is produced, and
- a stage of stimulation of cell proliferation consisting in feeding alternating current or direct current to the electrical contacts (8) of the bracket (4) in the bioreactor (1).

9. Production process of adherent cell cultures, according to claim 8, that further comprises a stage of cell proliferation stimulation consisting in the production of air bubbles underneath each tray (5) by means of the air bubble generators (9).
